(19) Europäisches Patentamt
European Patent Office
Office européen des brevets

(11) Veröffentlichungsnummer: **0 285 946 B1**

# (12) EUROPÄISCHE PATENTSCHRIFT

(45) Veröffentlichungstag der Patentschrift: **21.11.91**

(51) Int. Cl.[5]: **C07C 311/16**, C08K 5/43

(21) Anmeldenummer: **88104947.2**

(22) Anmeldetag: **28.03.88**

## (54) N-Alkyl-benzolsulfonamide.

(30) Priorität: **07.04.87 DE 3711689**

(43) Veröffentlichungstag der Anmeldung:
**12.10.88 Patentblatt 88/41**

(45) Bekanntmachung des Hinweises auf die Patenterteilung:
**21.11.91 Patentblatt 91/47**

(84) Benannte Vertragsstaaten:
**BE CH DE FR GB IT LI NL**

(56) Entgegenhaltungen:
**EP-A- 0 007 623**
**EP-A- 0 069 278**
**DE-A- 2 617 180**
**US-A- 2 214 405**

(73) Patentinhaber: **BAYER AG**
**W-5090 Leverkusen 1 Bayerwerk(DE)**

(72) Erfinder: **Schössler, Willi, Dr.**
**Hufelandstrasse 16**
**W-5000 Köln 80(DE)**

EP 0 285 946 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft N-Alkyl-benzolsulfonamide, deren Alkylgruppe offenkettiges $C_1$-$C_{12}$-Alkyl oder $C_5$-$C_7$-Cycloalkyl ist und deren Benzolkern durch $C_1$-$C_4$-Alkyl substituiert sein kann, mit einem Gehalt von 10 - 1000 ppm einer oder mehrerer 2-Mercapto-benzimidazol-Verbindungen der Formel

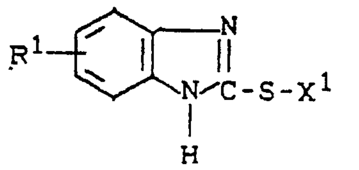

(I),

in der

$R^1$ Wasserstoff oder eine $C_1$-$C_4$-Alkylgruppe und

$X^1$ Wasserstoff oder 1 Äquivalent der Metalle Zn, Cd oder Mn(II) bedeuten.

Die Erfindung betrifft also Gemische von 10 - 1000 ppm der genannten 2-Mercapto-benzimidazol-Verbindung(en), bezogen auf die Gesamtmenge des Gemisches, und dem Rest zu 100 % der Gesamtmenge des Gemisches an den genannten N-Alkyl-benzolsulfonamiden.

Die N-Alkyl-benzolsulfonamide stellen hierbei reine Substanzen dar, wie sie aus den weiter unten beschriebenen Herstellungsverfahren und anschließender Aufarbeitung bzw. Reinigung erhalten werden. Die Reinheit beträgt regelmäßig 99,5 % oder darüber, in vielen Fällen 99,9 %, Restliche geringe Verunreinigungen stammen aus dem Herstellungsverfahren.

Die genannten 2-Mercaptro-benzimidazol-Verbindungen stellen Stabilisatoren der N-Alkyl-benzolsulfonamide, beispielsweise gegen eine thermisch hervorgerufene Beeinträchtigung, dar. Die Erfindung betrifft also durch die genannten 2-Mercapto-benzimidazol-Verbindungen stabilisierte N-Alkyl-benzolsulfonamide der genannten Art.

N-Alkyl-Gruppen der Benzolsulfonamide sind beispielsweise offenkettige mit 1 - 12 und cyclische mit 5 - 7 C-Atomen, wie Methyl, Ethyl, Propyl, i-Propyl, n-Butyl, i-Butyl, die geradkettigen oder verzweigten Amyle, Hexyle, Heptyle, Octyle, Decyle oder Dodecyle, sowie Cyclopentyl, Methylcyclopentyl, Cyclohexyl und Methylcyclohexyl. Die N-Alkylgruppen sind in bevorzugter Weise offenkettige mit 1 - 8 C-Atomen beziehungsweise das Cyclohexyl; in besonders bevorzugter Weise sind es offenkettige mit 1 - 6 C-Atomen beziehungsweise das Cyclohexyl.

Weitere bevorzugte N-Alkyl-Gruppen sind solche mit 4 - 8 C-Atomen, Ganz besonders bevorzugte N-Alkyl-Gruppen sind n-Butyl, n-Hexyl und 2-Ethyl-hexyl.

$C_1$-$C_4$-Alkyl im Benzolkern der Sulfonamide bzw. in den Benzimidazol-Verbindungen sind unabhängig voneinander beispielsweise Methyl, Ethyl, Propyl, i-Propyl, Butyl oder i-Butyl, bevorzugt Methyl oder Ethyl, besonders bevorzugt Methyl. Der Benzolkern der Sulfonamide trägt in ganz besonders bevorzugter Weise keine Substituenten.

Metallkationen sind Beispielsweise solche von Zn, Cd oder Mn(II), bevorzugt von Zn oder Mn(II), besonders bevorzugt von Zn.

In bevorzugter Weise seien 2-Mercapto-benzimidazol-Verbindungen der Formel

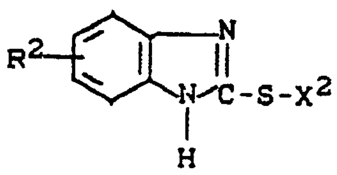

(II)

genannt, in der

$R^2$ Wasserstoff, Methyl oder Ethyl und

$X^2$ Wasserstoff oder 1 Äquivalent der Metalle Zn oder Mn(II) bedeuten.

In besonders bevorzugter Weise seien 2-Mercapto-benzimidazol-Verbindungen der Formel

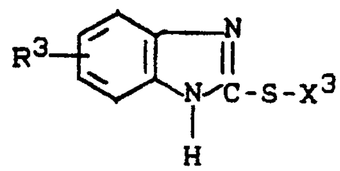

(III)

genannt, in der

$R^3$ Wasserstoff oder Methyl und

$X^3$ Wasserstoff oder $\frac{1}{2}$ $Zn^{2+}$ bedeuten.

Verbindungen der Formel (I), (II) und (III) sind beispielsweise: 2-Mercapto-4-methyl-benzimidazol, 2-Mercapto-5-methyl-benzimidazol, das Isomerengemisch aus 2-Mercapto-4-methyl- und 2-Mercapto-5-methyl-benzimidazol, 2-Mercapto-benzimidazol oder deren Zinksalze, bevorzugt das Isomerengemisch aus 2-Mercapto-4-methyl-und 2-Mercapto-5-methyl-benzimidazol und 2-Mercapto-benzimidazol, besonders bevorzugt 2-Mercapto-benzimidazol.

Die 2-Mercapto-benzimidazol-Verbingung(en) liegen in den erfindungsgemäßen stabilisierten N-Alkyl-benzolsulfonamiden in einer Menge von 10 - 1000, bevorzugt 15 - 300, besonders bevorzugt 15 - 200 ppm, bezogen auf die Gesamtmenge des Gemisches aus den genannten 2-Mercapto-benzimidazol-Verbindungen und den genannten N-Alkyl-benzolsulfonamiden, vor. Die stabilisierende Wirkung ist vielfach bereits ausreichend im Bereich von 15 - 100, sogar 15 - 50 ppm gegeben. Selbstverständlich können auch größere Mengen als 1000 ppm eingesetzt werden, jedoch zeigen sie keine zusätzliche Wirksamkeit.

Die Mischungsbestandteile der erfindungsgemäß stabilisierten Mischung sind bekannt und können nach bekannten Verfahren hergestellt werden. So ist es allgemein bekannt, Sulfonsäureamide durch Kondensation der Sulfonsäurechloride mit Aminen in Wasser oder in einem inerten Lösungsmittel (beispielsweise Benzol oder Aceton) in Gegenwart eines säurebindenden Mittels (beispielsweise Natronlauge, Natriumhydrogencarbonat, Calciumcarbonat, Natriumsulfit, Natriumacetat in Eisessig, einem zweiten Mol des Amins, Pyridin oder anderen) darzustellen (Ullmanns Encyclopädie der technischen Chemie, Band 8 (1974), S. 418, linke Spalte, 3. Abschnitt und Houben-Weyl, Methoden der organischen Chemie, Band 9 (1955), S. 609 - 616). Im Rahmen dieser allgemeinen Kenntnis kann beispielsweise N-n-Butyl-benzolsulfonamid aus Benzolsulfochlorid und n-Butylamin in Gegenwart von Alkali (Beilstein, Band 11 (H), S. 41) oder aus Benzolsulfochlorid und 2 Mol Butylamin in Ethanol (Beilstein, Band 11 (E III), S. 54; Rec. Trav. Chim. Pays-Bas, Band 50 (1931), S. 52, 1. Abschnitt) hergestellt werden.

Zur Aufarbeitung flüssiger Sulfonamide können diese, gegebenenfalls in Form einer Lösung in einem organischen Lösungsmittel wie Benzol, mit Wasser gewaschen, mit einem Trockenmittel (beispielsweise Natriumsulfat) getrocknet, filtriert und anschließend destilliert werden. Das Destillat kann gegebenenfalls mit Aktivkohlen, wie Norit, behandelt und nochmals filtriert werden (Ind. Eng. Chem. Band 46 (1954), S. 587 und 590). Des weiteren können flüssige Sulfonamide nach einer Herstellung im wäßrigen System als Ölschicht abgetrennt werden. Diese Ölschicht kann zur Entfernung von Wasser im Vakuum erwärmt werden, woraufhin das Öl von sehr wenig anorganischem Salz (beispielsweise Natriumchlorid, wenn in Gegenwart von NaOH oder $NaHCO_3$ gearbeitet wurde) abgesaugt wird (Houben-Weyl, Methoden der organischen Chemie, Band 9 (1955), S. 610, vorletzter Abschnitt). Bei der Entfernung des Wassers kann gegebenenfalls zusätzlich ein Adsorptionsmittel, beispielsweise Aktivkohle oder Bleicherde, eingesetzt werden. Schließlich ist eine Destillation des rohen Sulfonamids unter vermindertem Druck möglich. Nach diesen Verfahren können farblose bis schwach gelblich gefärbte N-Alkyl-benzolsulfonamide mit Hazen-Farbzahlen von (5 bis 15 erhalten werden. Sulfonamide, deren Erstarrungspunkte oberhalb der Raumtemperatur, jedoch unterhalb des Siedepunktes von Wasser liegen, können in gleicher Art als Öle aufgearbeitet werden, wenn man die Arbeitstemperatur entsprechend anpaßt.

Feste Sulfonamide können in bekannter Weise nach den für Feststoffe bekannten Methoden aufgearbeitet werden.

Die im erfindungsgemäßen Gemisch vorhandenen 2-Mercapto-benzimidazol-Verbindungen sind ebenfalls bekannt und beispielsweise dadurch herstellbar, daß man ein gegebenenfalls substituiertes o-Phenylendiamin mit Schwefelkohlenstoff bei erhöhter Temperatur unter Cyclisierung und $H_2S$-Abspaltung umsetzt. Die Metallverbindungen erhält man durch weitere Umsetzung mit geeigneten Metallsalzen (Ullmanns Enzyklopädie der Technischen Chemie, 4. Auflage, Band 23 (1983), S. 193, 195 und 196).

Die erfindungsgemäßen N-Alkyl-benzolsulfonamide, deren Alkylgruppe offenkettiges $C_1$-$C_{12}$-Alkyl oder $C_5$-$C_7$-Cycloalkyl ist und deren Benzolkern durch $C_1$-$C_4$-Alkyl substituiert sein kann, mit einem Gehalt von 10 - 1000 ppm einer oder mehrerer 2-Mercapto-benzimidazol-Verbindungen der Formel

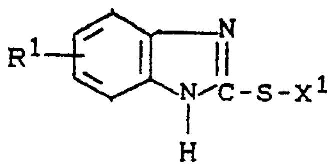

(I),

in der

$R^1$ Wasserstoff oder eine $C_1$-$C_4$-Alkylgruppe und

$X^1$ Wasserstoff oder 1 Äquivalent der Metalle Zn, Cd oder Mn(II) bedeuten,

können beispielsweise so hergestellt werden, daß man den in an sich üblicher Weise aus Benzolsulfochloriden, deren Benzolkern durch $C_1$-$C_4$-Alkyl substituiert sein kann, und $C_1$-$C_{12}$-Alkylaminen oder $C_5$-$C_7$-Cycloalkylaminen hergestellten N-Alkyl-benzolsulfonamiden die genannte Menge der 2-Mercapto-benzimidazol-Verbindung(en) zusetzt und homogen vermischt oder daß man solchen N-Alkyl-benzolsulfonamiden vor oder während ihrer Aufarbeitung nach der genannten Herstellung eine solche Menge einer oder mehrerer der 2-Mercapto-benzimidazol-Verbindung(en) zusetzt, daß unter Berücksichtigung der Verluste im Aufarbeitungsverfahren die genannte Menge der 2-Mercapto-benzimidazol-Verbindung(en) in den N-Alkyl-benzolsulfonamiden verbleibt.

Mann kann demnach 2-Mercapto-benzimidazol-Verbindungen der Formeln (I), (II) und/oder (III) den rohen N-Alkylbenzolsulfonamiden beispielsweise vor der Entfernung des Wassers zusetzen, wenn die Herstellung in einem wäßrigen System erfolgte oder eine Wäsche mit Wasser durchgeführt wurde, sodann in Anwesenheit von Adsorptionsmitteln das Wasser im Vakuum entfernen und anschließend filtrieren. Man kann die 2-Mercapto-benzimidazol-Verbindungen jedoch auch den N-Alkylbenzolsulfonamiden zusetzen, nachdem diese unter vermindertem Druck destilliert worden waren.

Hierbei können die 2-Mercapto-benzimidazol-Verbindungen in reiner Form oder als höherkonzentrierte Lösung in dem zu stabilisierenden N-Alkyl-benzolsulfonamid zugegeben werden. Für den Fall, daß im Rahmen der Aufarbeitung der rohen N-Alkyl-benzolsulfonamide von deren Lösung in einem organischen Lösungsmittel ausgegangen wird (beispielsweise in Benzol oder Aceton) können die 2-Mercapto-benzimidazol-Verbindungen auch in Form einer Lösung in einem solchen organischen Lösungsmittel zugesetzt werden, woraufhin die weiter oben beschriebenen Aufarbeitungsschritte durchgeführt werden.

N-Alkyl-benzolsulfonamide als solche, d.h. nicht in Form der erfindungsgemäßen, oben beschriebenen Mischung, sind bereits als Weichmacher für Polymere mit polaren Gruppen eingesetzt worden (Ind. Eng. Chem., Band 46 (1954), S. 590, rechte Spalte, 2. und 3. Absatz). Hierbei wurde festgestellt, daß mit N-Alkyl-benzolsulfonamiden weichgemachte Polymere beim Tempern bei 160 °C sich sehr unterschiedlich verfärbten. Eine genaue Abhängigkeit von der N-Alkylgruppe konnte nicht ermittelt werden.

Auch die bei den oben beschriebenen Aufarbeitungsmethoden erhaltenen N-Alkyl-benzolsulfonamide weisen ohne erkennbaren Grund und ohne Abhängigkeit von der einzelnen Aufarbeitungsvariante sehr unterschiedliche thermische Stabilitäten auf. Die unzureichende thermische Stabilität äußert sich in einer starken Verfärbung des getemperten N-Alkyl-benzolsulfonamids und macht dieses für die Herstellung farbloser weichgemachter Polymerer ungeeignet (siehe Vergleichsbeispiele im Rahmen der Ausführungsbeispiele).

Andererseits ist es bekannt, N-Alkyl-benzolsulfonamide, beispielsweise N-n-Butyl-benzolsulfonamid, als Weichmacher für Polymere mit polaren Gruppen, wie beispielsweise Nitrocellulose, Acetylcellulose oder Polyamide, einzusetzen (Ullmanns Enzyklopädie der Technischen Chemie. 4. Aufl. Bd. 24, (1983), S. 371 und 372). Beispielsweise verleiht N-n-Butyl-benzolsulfonamid Polyamiden eine höhere Elastizität, wenn sie zu Formteilen, wie Schuhsohlen, Schuhabsätzen und anderen technischen Teilen verarbeitet werden. Solche mit N-n-Butyl-benzolsulfonamid weichgemachten Polyamid-Formteile bleiben auch bei völliger Austrocknung geschmeidig und verspröden nicht, wie dies bei ausschließlich mit Wasser elastifizierten Polyamid-Formteilen beobachtet wird. Solche Polyamid-Formteile sollen unabhängig von ihrer Herstellung bei erhöhter Temperatur durch Spritzguß oder Extrusion und unabhängig vom Einsatz von N-Alkyl-benzolsulfonamiden, denen eine etwaige thermische Instabilität zunächst nicht anzusehen ist, stets als klare farblose Produkte entstehen. Hierzu sind stets gleichmäßig thermostabile N-Alkyl-benzolsulfonamide sehr wünschenswert.

Es hat nicht an eigenen Versuchen gefehlt, N-Alkyl-benzolsulfonamide in gleichmäßig thermostabiler Form herzustellen. So wurden beispielsweise Zusätze von 4,4'-Dihydroxydiphenyl, 2,2-Bis-(4-hydroxyphenyl)-propan, 2,2-Bis-(4-hydroxycyclohexyl)-propan, Octadecyl-3-(3,5-di-tert.-butyl-4-hydroxyphenyl)-propionat, Pentaerythrityl-tetrakis-[3-(3,5-di-tert.-butyl-4-hydroxyphenyl)-propionat] und 2,6-Di-tert.-butyl-4-methylphenol auch in größeren Mengen angewandet, ohne ausreichende thermostabilisierende Wirkung zu erzielen.

Die aus diesen Versuchen hervorgegangenen N-Alkyl-benzolsulfonamide waren daher für den Einsatz als Weichmacher ungeeignet. Die Wirksamkeit des erfindungsgemäßen Zusatzes von 2-Mercapto-benzimidazol-Verbindungen in den genannten kleinen Mengen ist daher überaus überraschend.

Die Erfindung betrifft weiterhin die Verwendung der erfindungsgemäß stabilisierten N-Alkyl-benzolsulfonamide als Weichmacher für Polymere mit polaren Gruppen, beispielsweise als Weichmacher für Nitrocellulose, Acetylcellulose, Celluloseacetopropionat, Celluloseacetobutyrat, Polyvinylchlorid, Polyester, Polyamide und ähnliche. Insbesondere betrifft die Erfindung die Verwendung der erfindungsgemäß stabilisierten N-Alkyl-benzolsulfonamide als Weichmacher für Polyamide.

Die stabilisierten N-Alkyl-benzolsulfonamide (d.h. die erfindungsgemäße Mischung aus den genannten N-Alkyl-benzolsulfonamiden und den ebenfalls genannten 2-Mercapto-benzimidazol-Verbindungen) können bei ihrer Verwendung gegebenenfalls gemeinsam mit anderen Zusätzen, wie Stabilisatoren für das Polymer, Farbmitteln und anderen bei der Polymer-Verarbeitung üblichen Zusätzen, eingesetzt werden. In solchen Fällen handelt es sich bei der erfindungsgemäßen Mischung um einen Gemischbestandteil eines größeren Gemisches.

Die Überprüfung der thermischen Stabilität von Weichmachern durch Überprüfung von durch Spritzguß oder Extrusion erhaltenen weichgemachten Polymeren ist umständlich und wegen des Einflusses von Parametern der dabei eingesetzten Maschinen sowie des Einflusses der wechselnden Qualität des Polymeren unsicher. Es ist daher sicherer und besser reproduzierbar, den Weichmacher allein zu tempern und seine qualität unabhängig von den anderen genannten Einflüssen festzustellen. Hierbei werden Proben des Weichmachers während unterschiedlicher Zeiten und bei unterschiedlichen Temperaturen erhitzt und anschließend ihre farbliche Veränderung festgestellt. Diese Zeiten und Temperaturen werden im allgemeinen den Verarbeitungsbedingungen der weichgemachten Polymeren, gegebenenfalls einschließlich geeigneter Sicherheitszuschläge, angepaßt.

Die farbliche Veränderung der erhitzten Proben wurde in den folgenden Beispielen mit Hilfe der bis zum Wert 500 definierten Hazen-Farbskala gemessen und dargestellt.

Bei den Vergleichsbeispielen ergaben sich vielfach Werte, die höher als 500 Hazen waren und damit außerhalb der Skala lagen. Da nach Analytical Chem., Vol. 49 (1977), S. 524 und 525 ein Zusammenhang zwischen spektrophotometrischen ermittelten Werten und der Hazen-Skala besteht, wurde in solchen Fällen die Extinktion bei 450 nm (Küvette D = 1 cm, Vergleichsmessung gegen die nicht erhitzte Probe) gemessen. Zur Einordnung solcher außerhalb der Hazen-Skala befindlicher Werte wurden auch an einigen Proben, die innerhalb der Hazen-Skala lagen, vergleichsweise die Extinktionen gemessen (innerhalb der Beispiele in Klammern angegeben).

Beispiel 1

N-n-Butyl-benzolsulfonamid wurde in bekannter Weise durch Kondensation von Benzolsulfochlorid und n-Butylamin in Anwesenheit von Wasser und Natronlauge hergestellt. Nach vollständigem Umsatz des Benzolsulfochlorids wurde das entstandene Öl von der wäßrigen Phase abgetrennt, nochmals mit Wasser gewaschen und wiederum von der wäßrigen Phase getrennt. Das Rohprodukt wurde in einer Glasapparatur mit je 1 Gew.-% A-Khole und Bleicherde als Adsorptionsmittel versetzt, zur Entfernung des Wassers im Vakuum erwärmt und anschließend filtriert. Das erhaltene N-n-Butyl-benzolsulfonamid hatte eine Reinheit von 99,9 % und eine Farbzahl von 5 Hazen.

Dieses N-n-Butyl-benzolsulfonamid wurde unter Rühren mit 50 bzw. 100 ppm 2-Mercapto-benzimidazol versetzt und anschließend während der in Tab. 1 angegebenen Zeiten auf die angegebenen Temperaturen erhitzt. Zum Vergleich wurde ein N-n-Butyl-benzolsulfonamid ohne einen Zusatz erhitzt. Die Hazen-Farbzahlen und einige Extinktionswerte der getemperten Proben wurden bestimmt (Tab. 1).

Tab. 1 (Beispiel 1): Hazen-Farbzahlen und Extinktionswerte (in Klammern) von getempertem N-n-Butylbenzolsulfonamid

| Nr. | Menge an 2-Mercapto-benzimidazol | 160° C 2Std. | 160° C 3Std. | 170° C 1Std. | 170° C 2Std. | 170° C 3Std. | 190° C 1Std. |
|---|---|---|---|---|---|---|---|
| 1a | 100 ppm | 10 | 15 | 10 | 30 | 60 | 55 |
| 1b | 50 ppm | 30 | 50 | 25 | 80 (0,018) | 110 (0,027) | 90 (0,020) |
| 1c* | ohne | 330 | 500 | 300 | >500 (0,185) | >500 (0,275) | >500 (0,262) |

* nicht erfindungsgemäßer Vergleichsversuch

Beispiel 2

Ein ähnlich wie in Beispiel 1 erhaltenes N-n-Butyl-benzolsulfonamid wurde mit einer Reinheit von 99,9 % und einer Farbzahl von <5 Hazen erhalten. Dieses N-n-Butyl-benzolsulfonamid wurde unter Rühren mit

10 bis 50 ppm 2-Mercapto-benzimidazol versetzt und anschließend auf 170 °C erhitzt. Nach 0,25 und 1 Std, wurden die Farbzahlen ermittelt. Zum Vergleich wurde die Temperstabilität des N-n-Butyl-benzolsulfonamids ohne Zusatz bestimmt. Alle Werte sind in Tab. 2 aufgeführt.

Tab. 2 (Beispiel 2): Hazen-Farbzahlen von bei 170° C getempertem N-n-Butyl-benzolsulfonamid

| Nr. | Menge an 2-Mercapto-benzimidazol | 0,25 Std. | 1 Std. |
|---|---|---|---|
| 2a | 50 ppm | <5 | 5 |
| 2b | 25 ppm | <5 | 5 |
| 2c | 20 ppm | <5 | 5 |
| 2d | 10 ppm | 5 | 10 |
| 2e* | ohne | 130 | 400 |

* nicht erfindungsgemäßer Vergleichsversuch

Beispiel 3

Ein ähnlich wie in Beispiel 1 erhaltenes N-n-Butyl-benzolsulfonamid mit einer Reinheit von 99,9 % und einer Farbzahl von <5 Hazen wurde unter Rühren mit jeweils 20 ppm 2-Mercapto-benzimidazol (Vulkanox MB®, Handelsprodukt der Firma Bayer AG), dem Isomerengemisch von 4-Methyl- und 5-Methyl-2-mercapto-benzimidazol (Vulkanox MB-2, Handelprodukt der Firma Bayer AG) bzw. dem Zinksalz des genannten Isomerengemischs (Vulkanox ZMB-2®, Handelsprodukt der Firma Bayer AG) versetzt und anschließend auf 170 °C erhitzt. Nach 1 Stunde Temperung wurden die Farbzahlen und einige Extinktionswerte ermittelt. Zum Vergleich wurde die Temperstabilität des N-n-Butyl-benzolsulfonamids ohne Zusatz bestimmt. Alle Werte sind in Tab. 3 aufgeführt.

Tab. 3 (Beispiel 3): Hazen-Farbzahlen und Extinkttionswerte (in Klammern) von getempertem N-n-Butyl-benzolsulfonamid (1 Std., 170° C) mit je 20 ppm verschiedener Zusätze.

| Nr. | Zusatz | Farbzahl |
|---|---|---|
| 3a | Vulkanox MB® | 15 |
| 3b | Vulkanox MB-2® | 50 |
| 3c | Vulkanox ZMB-2® | 55 (0,013) |
| 3d* | ohne | >500 (0,160) |

* nicht erfindungsgemäßer Vergleichsversuch

Mit den Mn(II)-Salzen des 2-Mercapto-benzimidazols und des genannten Isomerengemisches wurden vergleichbare Ergebnisse wie in Beispiel 3 c erhalten. Die große Schwankung der Hazen-Farbzahl des getemperten N-n-Butyl-benzolsulfonamids ohne Zusatz zeigt nicht aufgeklärte Zufälligkeiten bei fehlender Stabilisierung.

Beispiel 4

Es wurde wie in Beispiel 1 beschrieben verfahren, jedoch wurde die Reinigung des rohen N-n-Butyl-benzolsulfonamids in einer V4A-Apparatur durchgeführt. Das erhaltene reine Produkt hatte eine Reinheit von 99,9 % und eine Farbzahl von 5 Hazen. Dieses reine N-n-Butyl-benzolsulfonamid wurde unter Rühren mit 10 bis 1000 ppm 2-Mercapto-benzimidazol versetzt und anschließend auf 170° C erhitzt. Nach 0,25 und 1 Std. wurden die Farbzahlen und einige Extinktionswerte ermittelt. Zum Vergleich wurde das so gereinigte N-n-Butyl-benzolsulfonamid ohne Zusatz getempert. Alle Werte sind in Tab. 4 aufgeführt.

Tab. 4 (Beispiel 4): Hazen-Farbzahlen und Extinktionswerte (in Klammern) von bei 170° C getempertem N-n-Butyl-benzolsulfonamid

| Nr. | Menge an 2-Mercapto-benzimidazol | 0,25 Std. | 1 Std. |
|---|---|---|---|
| 4a | 1000 ppm | <5 | 20 |
| 4b | 200 ppm | <5 | 30 |
| 4c | 100 ppm | <5 | 35 |
| 4d | 50 ppm | 10 | 35 |
| 4e | 10 ppm | 10 | 150 (0,037) |
| 4f* | ohne | 120 | > 500 (0,390) |

* nicht erfindungsgemäßer Vergleichsversuch

Beispiel 5

Es wurde wie in Beispiel 1 beschrieben verfahren, jedoch wurde das 2-Mercapto-benzimidazol in Mengen von 100 bis 400 ppm, bezogen auf eingesetztes Rohprodukt, bereits vor der beschriebenen Reinigung zugesetzt. Man erhielt Produkte mit Reinheiten von 99,9 % und Farbzahlen von <5 Hazen. Die erhaltenen stabilisierten N-n-Butyl-benzolsulfonamide wurden auf 170° C erhitzt. Nach 0,25 und 1 Std. wurden die Farbzahlen ermittelt (Tab. 5).

Tab. 5 (Beispiel 5): Hazen-Farbzahlen von bei 170° C getempertem N-n-Butyl-benzolsulfonamid

| Nr. | 2-Mercapto-benzimidazol im Rohprodukt | 2-Mercapto-benzimidazol im Endprodukt | 0,25 Std. | 1 Std. |
|---|---|---|---|---|
| 5a | 400 ppm | ca. 200 ppm | <5 | 5 |
| 5b | 200 ppm | ca. 45 ppm | 5 | 30 |
| 5c | 100 ppm | ca. 12 ppm | 5 | 120 |

Beispiel 6

Es wurde wie in Beispiel 1 beschrieben verfahren, jedoch erfolgte die Reinigung des N-n-Butyl-benzolsulfonamids nach der zweiten Phasentrennung durch direkte fraktionierte Destillation im Vakuum in einer Glasapparatur, Man erhielt ein Produkt mit einer Reinheit von 99,9 % und einer Farbzahl von 15 Hazen. Das destillierte N-n-Butyl-benzolsulfonamid wurde unter Rühren mit 50 ppm 2-Mercapto-benzimidazol versetzt und anschließend während der in Tab. 6 angegebenen Zeiten auf die angegebenen Temperaturen erhitzt. Zum Vergleich wurde das N-n-Butyl-benzolsulfonamid ohne Zusatz erhitzt. Die Hazen-Farbzahlen und einige Extinktionswerte der getemperten Proben wurden bestimmt (Tab. 6).

**Tabelle 6:** (Beispiel 6): Hazen-Farbzahlen und Extinktionswerte (in Klammern) von getempertem N-n-Butyl-benzolsulfonamid

| Nr. | Menge an 2-Mercapto-benzimidazol | 160° C | | 170° C | | | 190° C |
|---|---|---|---|---|---|---|---|
| | | 3Std. | 4Std. | 1Std. | 2Std. | 3Std. | 1Std. |
| 6a | 50 ppm | 40 (0,010) | 60 (0,014) | 30 | 45 | 90 (0,021) | 80 (0,019) |
| 6b* | ohne | >500 (0,185) | >500 (0,260) | 350 | 500 | >500 (0,260) | >500 (0,162) |

* nicht erfindungsgemäßer Vergleichsversuch

Beispiel 7

N-Methyl-benzolsulfonamid wurde in Analogie zu Beispiel 1 durch Kondensation von Benzolsulfochlorid und wäßrigem Methylamin in Anwesenheit von Natronlauge hergestellt. Nach vollständigem Umsatz des Benzolsulfochlorids wurde das entstandene Öl wie in Beispiel 1 angegeben aufgearbeitet. Das erhaltene N-Methylbenzolsulfonamid hatte eine Reinheit von 99,8 % und eine Farbzahl von 5 Hazen.

Dieses N-Methyl-benzolsulfonamid wurde unter Rühren mit 50 ppm 2-Mercapto-benzimidazol versetzt und anschließend während der in Tab. 7 angegebenen Zeiten auf die angegebenen Temperaturen erhitzt. Zum Vergleich wurde ein N-Methyl-benzolsulfonamid ohne einen Zusatz erhitzt. Die Hazen-Farbzahlen der getemperten Proben wurden bestimmt (Tabelle 7).

**Tab. 7 (Beispiel 7): Hazen-Farbzahlen von getempertem N-Methyl-benzolsulfonamid**

| Nr. | Menge an 2-Mercapto-benzimidazol | 170° C | | 190° C |
|---|---|---|---|---|
| | | 1 Stunde | 3 Stunden | 1 Stunde |
| 7a | 50 ppm | 25 | 50 | 50 |
| 7b* | ohne | 90 | 150 | 150 |

* nicht erfindungsgemäßer Vergleichsversuch

Beispiel 8

N-n-Hexyl-benzolsulfonamid wurde in Analogie zu Beispiel 1 durch Kondensation von Benzolsulfochlorid und n-Hexylamin in Anwesenheit von Wasser und Natronlauge hergestellt. Nach vollständigem Umsatz des Benzolsulfochlorids wurde das entstandene Öl nach Ansäuern des Reaktionsgemisches auf pH 3 von der wäßrigen Phase abgetrennt, nochmals mit Wasser gewaschen, wobei ein pH von 7 eingestellt wurde und wiederum von der wäßrigen Phase getrennt. Das Rohprodukt wurde wie in Beispiel 1 angegeben aufgearbeitet. Das erhaltene N-n-Hexyl-benzolsulfonamid hatte eine Reinheit von 99,8 % und eine Farbzahl von 5 Hazen.

Dieses N-n-Hexyl-benzolsulfonamid wurde unter Rühren mit 50 bzw. 100 ppm 2-Mercapto-benzimidazol versetzt und anschließend auf 170° erhitzt. Nach 1 und 3 Stunden wurden die Farbzahlen und Extinktionswerte ermittelt. Zum Vergleich wurde die Temperstabilität des N-n-Hexyl-benzolsulfonamids ohne Zusatz bestimmt. Alle Werte sind in Tabelle 8 aufgeführt.

Tab. 8 (Beispiel 8): Hazen-Farbzahlen und Extinktionswerte (in Klammern) von bei 170° C getempertem N-n-Hexyl-benzolsulfonamid

| Nr. | Menge an 2-Mercapto-benzimidazol | 1 Stunde | 3 Std. |
|---|---|---|---|
| 8a | 100 ppm | 120 (0,030) | 270 (0,068) |
| 8b | 50 ppm | 440 (0,110) | >500 (0,215) |
| 8c* | ohne | >500 (0,335) | >>500 (1,021) |

* nicht erfindungsgemäßer Vergleichsversuch

Beispiel 9

N-2-Ethylhexyl-benzolsulfonamid wurde in Analogie zu Beispiel 8 durch Kondensation von Benzolsulfochlorid und 2-Ethylhexylamin in Anwesenheit von Wasser und Natronlauge hergestellt. Nach vollständigem Umsatz des Benzolsulfochlorids wurde das entstandene Öl wie in Beispiel 8 angegeben aufgearbeitet. Das erhaltene N-2-Ethylhexylbenzolsulfonamid hatte eine Reinheit von > 99,9 % und eine Farbzahl von 5 Hazen.

Dieses N-2-Ethylhexyl-benzolsulfonamid wurde unter Rühren mit 50 bzw. 100 ppm 2-Mercaptobenzimidazol versetzt und anschließend auf 170° C erhitzt. Nach 1 und 3 Stunden wurden die Farbzahlen und einige Extinktionswerte ermittelt. Zum Vergleich wurde die Temperstabilität des N-2-Ethylhexyl-benzolsulfonamids ohne Zusatz bestimmt. Alle Werte sind in Tabelle 9 aufgeführt.

Tab. 9 (Beispiel 9): Hazen-Farbzahlen und einige Extinktionswerte (in Klammern) von bei 170° C getempertem N-2-Ethylhexyl-benzolsulfonamid

| Nr. | Menge an 2-Mercapto-benzimidazol | 1 Stunde | 3 Std. |
|---|---|---|---|
| 9a | 100 ppm | 12 | 160 (0,040) |
| 9b | 50 ppm | 160 (0,040) | >500 (0,413) |
| 9c* | ohne | >500 (0,341) | >>500 (1,284) |

* nicht erfindungsgemäßer Vergleichsversuch

Beispiele 10 - 13

In analogie zu Beispiel 8 wurden aus Benzolsulfochlorid und Cyclohexylamin, 4-Methyl-cyclohexylamin, n-Decylamin bzw. n-Dodecylamin, die zugehörigen N-Alkyl- bzw. N-Cycloalkyl-benzolsulfonamide hergestellt. Ihre Gemische mit jeweils 200 ppm 2-Mercapto-benzimidazol zeigten ein sehr gutes Temperverhalten, verglichen mit den nicht stabilisierten Verbindungen.

## Patentansprüche

1. N-Alkyl-benzolsulfonamide, deren Alkylgruppe offenkettiges $C_1$-$C_{12}$-Alkyl oder $C_5$-$C_7$-Cycloalkyl ist und deren Benzolkern durch $C_1$-$C_4$-Alkyl substituiert sein kann, mit einem Gehalt von 10 bis 1000 ppm einer oder mehrerer 2-Mercapto-benzimidazol-Verbindungen der Formel

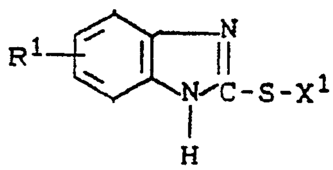

,

in der

$R^1$ Wasserstoff oder eine $C_1$-$C_4$-Alkylgruppe und
$X^1$ Wasserstoff oder 1 Äquivalent der Metalle Zn, Cd oder Mn(II) bedeuten.

2. N-Alkyl-benzolsulfonamide nach Anspruch 1, deren Alkylgruppe 1 - 8 C-Atome hat oder Cyclohexyl darstellt,

3. N-n-Butyl-benzolsulfonamid, N-n-Hexyl-benzolsulfonamid und N-2-Ethylhexyl-benzolsulfonamid nach Anspruch 1.

4. N-Alkyl-benzolsulfonamide nach Ansprüchen 1 bis 3 mit einem Gehalt an einer oder mehreren 2-Mercapto-benzimidazol-Verbindungen der Formel

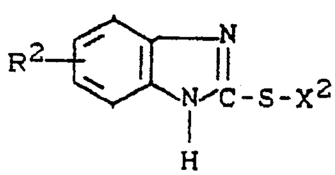

,

in der

$R^2$ Wasserstoff, Methyl oder Ethyl und
$X^2$ Wasserstoff oder 1 Äquivalent der Metalle Zn oder Mn(II) bedeuten.

5. N-Alkyl-benzolsulfonamide nach Ansprüchen 1 bis 4 mit einem Gehalt an einer oder mehreren 2-Mercapto-benzimidazol-Verbindungen der Formel

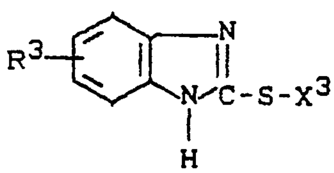

,

in der

$R^3$ Wasserstoff oder Methyl und
$X^3$ Wasserstoff oder $\frac{1}{2}$ $Zn^{2+}$ bedeuten.

6. N-Alkyl-benzolsulfonamide nach Ansprüchen 1 bis 5 mit einem Gehalt von 15 bis 300 ppm der 2-Mercapto-benzimidazol-Verbindung(en).

7. N-Alkyl-benzolsulfonamide nach Ansprüchen 1 bis 6 mit einem Gehalt von 15 - 200 ppm der 2-Mercapto-benzimidazol-Verbindung(en).

8. Verfahren zur Herstellung von N-Alkyl-benzolsulfonamiden, deren Alkylgruppe offenkettiges $C_1$-$C_{12}$-Alkyl oder $C_5$-$C_7$-Cycloalkyl ist und deren Benzolkern durch $C_1$-$C_4$-Alkyl substituiert sein kann, mit einem Gehalt von 10 bis 1000 ppm einer oder mehrerer 2-Mercapto-benzimidazol-Verbindungen der Formel

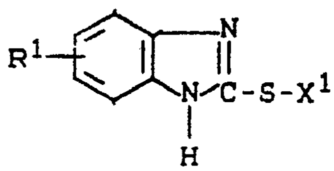
,

in der

$R^1$ Wasserstoff oder eine $C_1$-$C_4$-Alkylgruppe und
$X^1$ Wasserstoff oder 1 Äquivalent der Metalle Zn, Cd oder Mn(II) bedeuten,

dadurch gekennzeichnet, daß man den in an sich üblicher Weise aus Benzolsulfochloriden, deren Benzolkern durch $C_1$-$C_4$-Alkyl substituiert sein kann, und $C_1$-$C_{12}$-Alkylaminen oder $C_5$-$C_7$-Cycloalkylaminen hergestellten N-Alkyl-benzolsulfonamiden die genannte Menge der 2-Mercapto-benzimidazol-Verbindung(en) zusetzt und homogen vermischt

oder daß man solchen N-Alkyl-benzolsulfonamiden vor oder während ihrer Aufarbeitung nach der genannten Herstellung eine solche Menge einer oder mehrerer 2-Mercapto-benzimidazol-Verbindung-(en) zusetzt, daß unter Berücksichtigung der Verluste im Aufarbeitungsverfahren die genannte Menge der 2-Mercapto-benzimidazol-Verbindung(en) in den N-Alkyl-benzolsulfonamiden verbleibt.

9. Verwendung von N-Alkyl-benzolsulfonamiden, deren Alkylgruppe offenkettiges $C_1$-$C_{12}$-Alkyl oder $C_5$-$C_7$-Cycloalkyl ist und deren Benzolkern durch $C_1$-$C_4$-Alkyl substituiert sein kann, mit einem Gehalt von 10 bis 1000 ppm einer oder mehrerer 2-Mercapto-benzimidazol-Verbindungen der Formel

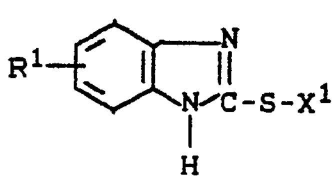
,

in der

$R^1$ Wasserstoff oder eine $C_1$-$C_4$-Alkylgruppe und
$X^1$ Wasserstoff oder 1 Äquivalent der Metalle Zn, Cd oder Mn(II) bedeuten,

als Weichmacher für Polymere mit polaren Gruppen.

10. Verwendung nach Anspruch 9 als Weichmacher für Polyamide.

**Claims**

1. N-Alkyl-benzenesulphonamides, the alkyl group of which is open-chain $C_1$-$C_{12}$-alkyl or $C_5$-$C_7$-cycloalkyl and the benzene nucleus of which can be substituted by $C_1$-$C_4$-alkyl, containing 10 to 1,000 ppm of one or more 2-mercapto-benzimidazole compounds of the formula

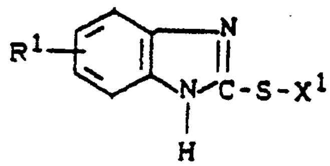

,

wherein

$R^1$ denotes hydrogen or a $C_1$-$C_4$-alkyl group and

$X^1$ denotes hydrogen or 1 equivalent of the metals Zn, Cd or Mn(II).

2. N-Alkyl-benzenesulphonamides according to Claim 1, the alkyl group of which has 1 - 8 C atoms or represents cyclohexyl.

3. N-n-Butyl-benzenesulphonamide, N-n-hexyl-benzenesulphonamide and N-2-ethylhexyl-benzenesulphonamide according to Claim 1.

4. N-Alkyl-benzenesulphonamides according to Claims 1 to 3, containing one or more 2-mercapto-benzimidazole compounds of the formula

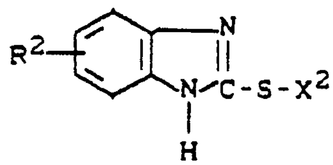

,

wherein

$R^2$ denotes hydrogen, methyl or ethyl and

$X^2$ denotes hydrogen or 1 equivalent of the metals Zn or Mn(II).

5. N-Alkyl-benzenesulphonamides according to Claims 1 to 4, containing one or more 2-mercapto-benzimidazole compounds of the formula

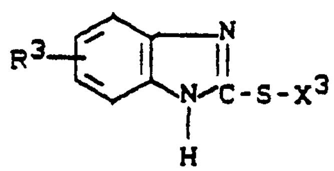

,

wherein

$R^3$ denotes hydrogen or methyl and

$X^3$ denotes hydrogen or 1/2 $Zn^{2+}$.

6. N-Alkyl-benzenesulphonamides according to Claims 1 to 5, containing 15 to 300 ppm of the 2-mercapto-benzimidazole compound(s).

7. N-Alkyl-benzenesulphonamides according to Claims 1 to 6, containing 15 - 200 ppm of the 2-mercapto-benzimidazole compound(s).

8. Process for the preparation of N-alkyl-benzenesulphonamides, the alkyl group of which is open-chain $C_1$-$C_{12}$-alkyl or $C_5$-$C_7$-cycloalkyl and the benzene nucleus of which can be substituted by $C_1$-$C_4$-alkyl, containing 10 to 1,000 ppm of one or more 2-mercapto-benzimidazole compounds of the formula

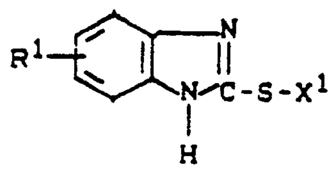
,

wherein

$R^1$ denotes hydrogen or a $C_1$-$C_4$-alkyl group and

$X^1$ denotes hydrogen or 1 equivalent of the metals Zn, Cd or Mn(II),

characterised in that the said quantity of the 2-mercapto-benzimidazole compound(s) is added to and homogeneously mixed with the N-alkyl-benzenesulphonamides prepared in the conventional manner from benzenesulphochlorides, the benzene nucleus of which can be substituted by $C_1$-$C_4$-alkyl, and $C_1$-$C_{12}$-alkylamines or $C_5$-$C_7$-cycloalkylamines,
or that such a quantity of one or more 2-mercapto-benzimidazole compound(s) is added to such N-alkyl-benzenesulphonamides before or during their working-up after the said preparation that, allowing for the losses in the working-up process, the said quantity of the 2-mercapto-benzimidazole compound-(s) remains in the N-alkyl-benzenesulphonamides.

9. Use of N-alkyl-benzenesulphonamides, the alkyl group of which is open-chain $C_1$-$C_{12}$-alkyl or $C_5$-$C_7$-cycloalkyl and the benzene nucleus of which can be substituted by $C_1$-$C_4$-alkyl, containing 10 to 1,000 ppm of one or more 2-mercapto-benzimidazole compounds of the formula

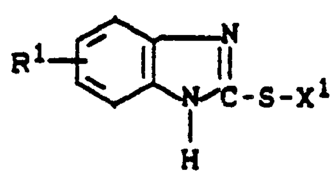
,

wherein

$R^1$ denotes hydrogen or a $C_1$-$C_4$-alkyl group and

$X^1$ denotes hydrogen or 1 equivalent of the metals Zn, Cd or Mn(II),

as a plasticiser for polymers having polar groups.

10. Use according to Claim 9 as a plasticiser for polyamides.

**Revendications**

1. N-alkylbenzène-sulfonamides dont le groupe alkyle consiste en un groupe alkyle acyclique en $C_1$-$C_{12}$ ou en un groupe cycloalkyle en $C_5$-$C_7$ et dont le noyau benzénique peut être substitué par des groupes alkyle en $C_1$-$C_4$, contenant de 10 à 1 000 ppm d'un ou plusieurs 2-mercaptobenzimidazoles de formule

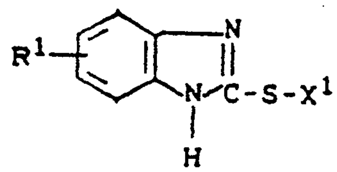

,

dans laquelle
$R^1$ représente l'hydrogène ou un groupe alkyle en $C_1$-$C_4$, et
$X^1$ représente l'hydrogène ou un équivalent des métaux Zn, Cd ou Mn(II).

2. N-alkylbenzène-sulfonamides selon la revendication 1, dont le groupe alkyle est un groupe alkyle en $C_1$-$C_8$ ou un groupe cyclohexyle.

3. Le N-n-butylbenzène-sulfonamide, le N-n-hexylbenzène-sulfonamide et le N-2-éthylhexylbenzène-sulfonamide selon la revendication 1.

4. N-alkylbenzène-sulfonamides selon les revendications 1 à 3, contenant un ou plusieurs 2-mercaptobenzimidazoles de formule

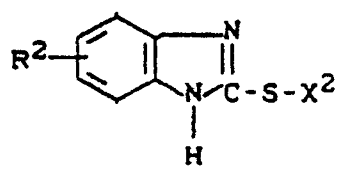

,

dans laquelle
$R^2$ représente l'hydrogène, un groupe méthyle ou éthyle, et
$X^2$ représente l'hydrogène ou un équivalent des métaux Zn ou Mn(II).

5. N-alkylbenzène-sulfonamides selon les revendications 1 à 4, contenant un ou plusieurs 2-mercaptobenzimidazoles de formule

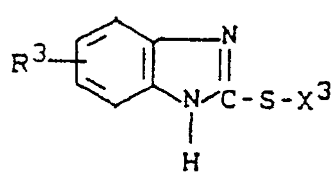

,

dans laquelle
$R^3$ représente l'hydrogène ou un groupe méthyle, et
$X^3$ représente l'hydrogène ou 1/2 $Zn^{+2}$.

6. N-alkylbenzène-sulfonamides selon les revendications 1 à 5, contenant 15 à 300 ppm de 2-mercaptobenzimidazole(s).

7. N-alkylbenzène-sulfonamides selon les revendications 1 à 6, contenant 15 à 200 ppm de 2-mercaptobenzimidazole(s).

8. Procédé de préparation de N-alkylbenzène-sulfonamides dont le groupe alkyle est un groupe alkyle acyclique en $C_1$-$C_{12}$ ou un groupe cycloalkyle en $C_5$-$C_7$ et dont le noyau benzénique peut être substitué par des groupes alkyle en $C_1$-$C_4$, contenant de 10 à 1 000 ppm d'un ou plusieurs 2-mercaptobenzimidazoles de formule

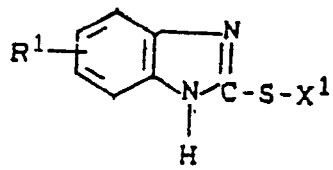

,

dans laquelle

$R^1$ représente l'hydrogène ou un groupe alkyle en $C_1$-$C_4$, et

$X^1$ représente l'hydrogène ou un équivalent des métaux Zn, Cd ou Mn(II),

caractérisé en ce que, à des N-alkylbenzène-sulfonamides préparés de la manière habituelle à partir de benzène-sulfochlorures dont le noyau benzénique peut être substitué par des groupes alkyle en $C_1$-$C_4$, et d'alkylamines en $C_1$-$C_{12}$ ou de cycloalkylamines en $C_5$-$C_7$, on ajoute la quantité indiquée des 2-mercaptobenzimidazole(s) et on mélange jusqu'à homogénéité,

ou bien en ce que, à ces N-alkylbenzène-sulfonamides, avant ou durant leur isolement après préparation comme décrit ci-dessus, on ajoute un ou plusieurs 2-mercaptobenzimidazole(s) en quantité telle que, tenu compte des pertes produites dans les opérations d'isolement, il subsiste dans les N-alkylbenzène-sulfonamides la quantité indiquée de 2-mercaptobenzimidazole(s).

9. Utilisation de N-alkylbenzène-sulfonamides dont le groupe alkyle est un groupe alkyle acyclique en $C_1$-$C_{12}$ ou un groupe cycloalkyle en $C_5$-$C_7$ et dont le noyau benzénique peut être substitué par des groupes alkyle en $C_1$-$C_4$, contenant de 10 à 1 000 ppm d'un ou plusieurs 2-mercaptobenzimidazole(s) de formule

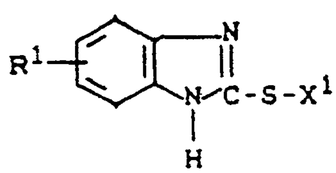

,

dans laquelle

$R^1$ représente l'hydrogène ou un groupe alkyle en $C_1$-$C_4$, et

$X^1$ représente l'hydrogène ou un équivalent des métaux Zn, Cd ou Mn(II),

en tant que plastifiants pour des polymères à groupes polaires.

10. Utilisation selon la revendication 9, en tant que plastifiants pour des polyamides.